Europäisches Patentamt

European Patent Office

Office européen des brevets

(11) Publication number: 0 343 885
A1

# EUROPEAN PATENT APPLICATION

(21) Application number: 89305125.0

(22) Date of filing: 19.05.89

(51) Int. Cl.⁴: **C12M 1/04** , **C12M 3/00**

(30) Priority: 19.05.88 IL 86442

(43) Date of publication of application:
29.11.89 Bulletin 89/48

(84) Designated Contracting States:
AT BE CH DE ES FR GB GR IT LI LU NL SE

(71) Applicant: **P.B. Ind. Plant Biotech Industries Ltd.**
**Mobile Post**
**Ashrat 25201(IL)**

(72) Inventor: **Kalfon, Abraham Rami**
**7 bis. Avenue Gounod**
**F-78290 Croissy s/Seine(FR)**

(74) Representative: **Lamb, John Baxter et al**
**MARKS & CLERK 57/60 Lincoln's Inn Fields**
**London WC2A 3LS(GB)**

(54) **Air lift fermentor formed from flexible plastic sheets.**

(57) An airlift fermenter comprising a sleeve formed of plastic film, first and second plastic welds formed across the sleeve to define a sealed fermentation enclosure and a divider panel formed of plastic film welded to the sleeve and disposed interiorly thereof and spatially separated from the first and second plastic welds, the sleeve and the divider panel being configured such that when the sleeve is disposed generally vertically and is inflated with a liquid, the divider panel is tensioned to lie taut and to generally bisect the volume of the sleeve.

EP 0 343 885 A1

## Air Lift Fermentor formed from Flexible Plastics Sheets

The present invention relates to fermenters and more particularly to airlift fermenters.

Air lift fermentors are often used for batch culture of shear sensitive cells and tissues, such as plant cells and mammalian cells. Batch fermentation processes are typically capital intensive and rely on sophisticated technology. In cases where market demand for a product is low and batch cycles are long, as in the culture of plant cells and tissues, high capital costs can preclude economical production of a product.

Some attempts have been made to reduce capital costs by the use of plastic fermentation vessels. An example of such fermenters is described in European Patent application 200792 to Markl which describes a fermenter formed of flexible plastic sheets clamped between two faceplates. The fermenter has the disadvantage that it must either be assembled under sterile conditions or sterilized in situ. Both sterilization procedures are relatively expensive.

Fermenters which are formed entirely of plastic sheets are known but they lack a center partition, which limits their usefulness. U.S. patent 4,668,632 describes a free standing plastic fermenter including a partition which can be radiation sterilized. However, its rigid construction makes it inconvenient and expensive to sterilize, ship and handle.

The present invention seeks to provide an improved, low cost airlift fermenter.

There is thus provided in accordance with a preferred embodiment of the present invention an airlift fermenter comprising a sleeve formed of plastic film, first and second plastic welds formed across the sleeve to define a sealed fermentation enclosure and a divider panel formed of plastic film welded to the sleeve and disposed interiorly thereof and spatially separated from the first and second plastic welds, the sleeve and the divider panel being configured such that when the sleeve is disposed generally vertically and is inflated with a liquid, the divider panel is tensioned to lie taut and to generally bisect the volume of the sleeve.

Further in accordance with a preferred embodiment of the invention, the first plastic weld also defines an upper end of the sleeve by which end the sleeve is supported from above in a hanging generally vertical orientation.

Additionally in accordance with a preferred embodiment of the invention a port which allows access to the interior of the fermenter without introducing contaiminants is sealingly welded to the sleeve.

Further in accordance with a preferred embodiment of the invention, pressurized gas is inserted into the sleeve at a location adjacent the bottom part of the divider panel. Preferably the pressurized gas is supplied by a compressor located outside of the sleeve via an gas tube extending through the port and terminating in a sparger which produces bubbles.

A magnet or ferromagnetic material may be associated with the sparger to permit desired orientation thereof by suitable positioning of a magnet or ferromagnetic material, as the case may be, exteriorly of the fermenter.

In accordance with a preferred embodiment of the invention, the entire fermenter is formed of radiation sterilizable materials.

The present invention will be understood and appreciated more fully from the following detailed description, taken in conjunction with the drawings in which:

Fig. 1 is a pictorial illustration of a airlift fermenter constructed and operative in accordance with a preferred embodiment of the present invention; and

Fig. 2 is a side sectional illustration of the airlift fermenter of Fig. 1.

## DETAILED DESCRIPTION OF A PREFERRED EMBODIMENT

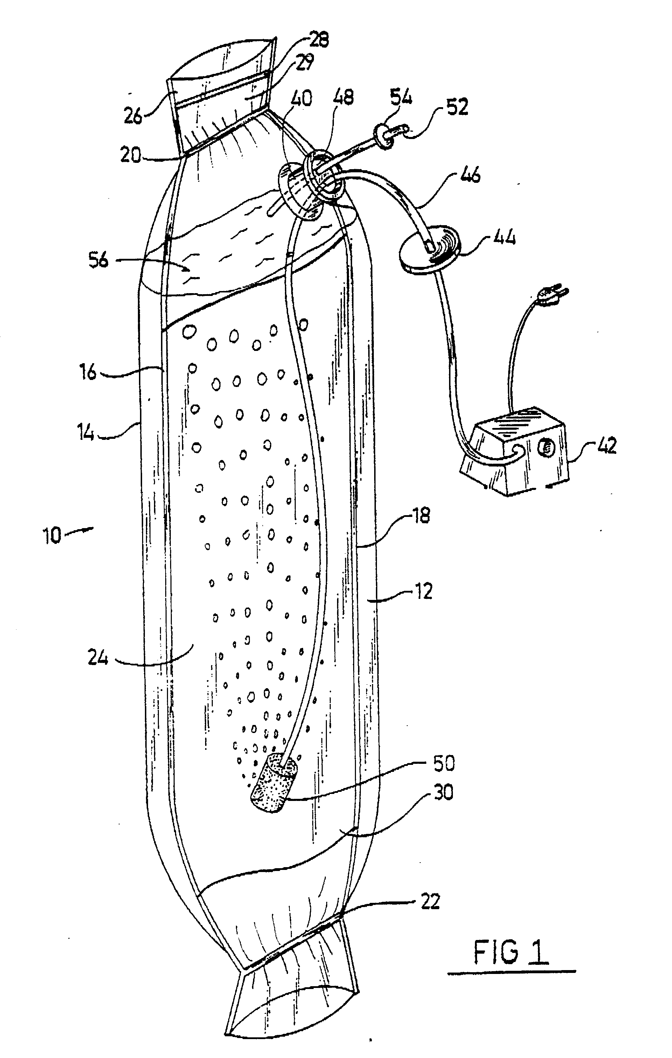

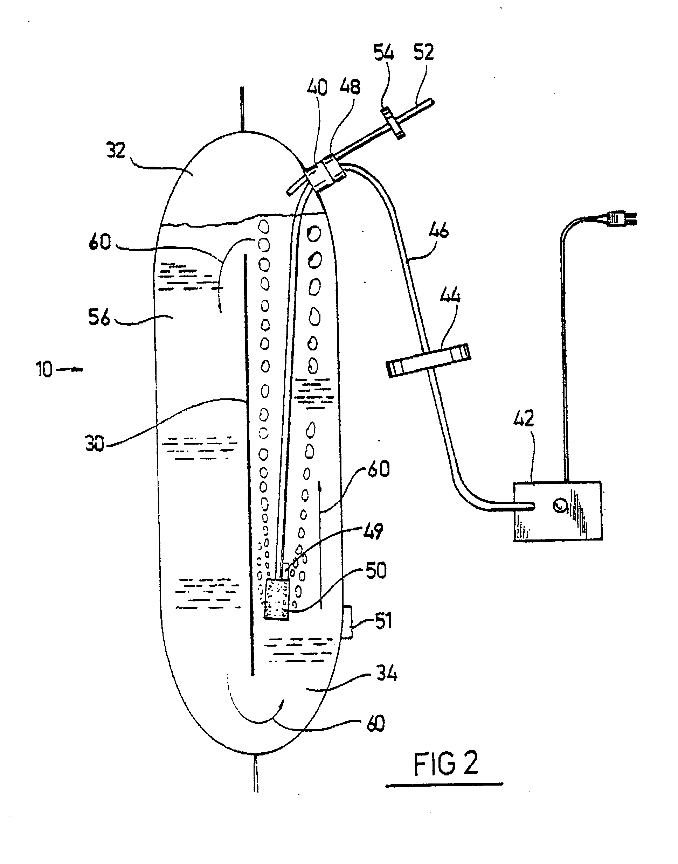

Reference is now made to Figs. 1 and 2, which show an airlift fermenter constructed and operative in accordance with a preferred embodiment of the present invention. The fermenter comprises a sleeve 10 typically formed of two clear flexible sheets, 12 and 14. Each sheet is typically a laminate which may be formed of a plurality of sheets of polyethylene or alternatively one sheet of polyethylene and one sheet of nylon joined together. Each laminate has a typical thickness of 100 microns. Sheets 12 and 14 are welded together along their respective side edges along weld lines 16 and 18. The sleeve 10 thus defined, is welded adjacent the top and bottom thereof along weld lines 20 and 22 to define a sealed enclosure 24.

The portion 26 of the sleeve 10 which lies above weld line 20 may be used for hanging support of the fermenter and may be formed with a second weld line 28 defining a transverse sleeve 29, through which a horizontal hanging rod (not shown) may extend for support of the fermenter. It is a particular feature of the present invention that end plates are not required for mounting or support of the fermenter.

A divider 30, also typically formed of a sheet of flexible clear plastic material, is arranged interiorly of the sleeve 10 and of the enclosure 24 and such that it does not extend to either of weld lines 20 or 22. Divider 30 is welded along its side edges to sleeve 10, preferably along weld lines 16 and 18. The welding of divider 30 to sleeve 10 preferably takes place in the same operation that joins sheets 12 and 14 together along weld lines 16 and 18.

The width of divider 30 is selected to be such that when enclosure 24 is inflated and assumes a generally circular cylindrical configuration, divider 30 is held taut and generally bisects the enclosure 24, while defining flow passages 32 and 34 respectively, above and below the divider, which permit liquid circulation between the two sides of the enclosure.

A port 40 is sealingly welded to an upper side wall of enclosure 24. Port 40, which may be provided with threading, permits sterile insertion and removal of materials to and from the enclosure 24.

A compressor 42 which may be a conventional compressor, such as that use in aquaria, provides a pressurized gas output via a filter 44 which removess contaminants from the gas and via tubing 46 which passes through a plug 48 that sealingly engages socket 40 in order to prevent the entry of contaminants to the interior of enclosure 24. Tubing 46 terminates in a sparger 50 which is preferably located adjacent the bottom portion of divider 30.

According to a preferred embodiment of the invention, a magnet or ferromagnetic material 49 may be associated with the sparger. Another magnet or ferromagnetic material 51 may be located outside the fermenter at a desired location, for desired placement of the sparger therewithin.

A pressure maintenance gas vent 52 associated with a bacteria proof filter 54 sealingly communicates with the interior of enclosure 24 above the liquid level via plug 48.

A liquid culture medium 56 is inserted into the enclosure 24 via socket 40 and partially fills the enclosure 24 approximately up to the level indicated, which lies above the top of divider 30. Biological material sought to be grown is added to the medium 56 and the supply of pressurized gas, such as air, from compressor 42 is provided, thus producing circulation of the liquid medium 56 and the biological material therein in directions illustrated by arrows 60.

It is a particular feature of the present invention that the entire fermenter enclosure is formed of conventional, low cost flexible plastic sheeting and is manufactured by low cost, conventional, mass production techniques. The port 40 and attachment thereof to the enclosure 24 are entirely conventional and produced by mass production techniques. Prior to sterilization and use, the fermenter can be folded or rolled down to an extremely small size for shipping and storage.

Cellular material may be inserted into enclosure 24 via port 40 by conventional sterile transfer techniques.

The temperature inside the enclosure 24 may be governed by partially submerging the fermenter in a liquid cooling bath.

It will be appreciated by persons skilled in the art that the present invention is not limited by what has been particularly shown and described hereinabove. Rather the scope of the present invention is defined only by the claims which follow:

## Claims

1. An airlift fermenter comprising:
a sleeve formed of plastic film;
first and second plastic welds formed across the sleeve to define a sealed fermentation enclosure; and
a divider panel formed of plastic film welded to the sleeve and disposed interiorly thereof and spatially separated from the first and second plastic welds, the sleeve and the divider panel being configured such that when the sleeve is disposed generally vertically and is inflated with a liquid, the divider panel is tensioned to lie taut and to generally bisect the volume of the sleeve.

2. A fermenter according to claim 1 and wherein said first plastic weld also defines an upper end of the sleeve by which the sleeve is supported from above in a hanging generally vertical orientation orientation.

3. A fermenter according to either of the preceding claims and also comprising a communication socket which is sealingly welded to the sleeve.

4. A fermenter according to claim 3 and also comprising means for inserting pressurized gas into the sleeve at a location adjacent the bottom part of the divider panel.

5. A fermenter according to claim 4 and wherein said means for inserting comprises a compressor located outside of the sleeve which produces a pressurized gas output via a gas tube extending through the communication socket and terminating in a sparger which produces bubbles.

6. A fermenter according to any of the preceding claims and being radiation sterilizable.

FIG 1

FIG 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 395 454 (CENTRE NATIONAL POUR L'EXPLORATION DES OCEANS) * Page 1, lines 6-17; page 2, line 2 - page 3, line 11; claims 1-3; figure 1 * --- | 1-6 | C 12 M 1/04 C 12 M 3/00 |
| A | US-A-3 184 395 (J.H. BREWER) * Column 2, lines 30-52; column 3, lines 3-5; column 3, line 73 - column 4, line 7; examples 1,2; claim 1; figures 1,3 * --- | 1,3,6 | |
| A | EP-A-0 222 529 (I.C.I.) * Column 1, lines 4-23; column 2, lines 36-49; column 4, lines 28-47; claim 8; figure 1 * ----- | 1,4,5 | |
| | | | TECHNICAL FIELDS SEARCHED (Int. Cl.4) |
| | | | C 12 M |

The present search report has been drawn up for all claims

| Place of search | Date of completion of the search | Examiner |
|---|---|---|
| THE HAGUE | 08-09-1989 | GROENENDIJK M.S.M. |